(19) Europäisches Patentamt
European Patent Office
Office européen des brevets

(11) Numéro de publication : **0 007 834**
**B1**

(12) # FASCICULE DE BREVET EUROPÉEN

(45) Date de publication du fascicule du brevet : **11.05.83**

(51) Int. Cl.³ : **C 07 C 99/00**, C 07 C101/02, C 07 B 29/00

(21) Numéro de dépôt : **79400462.2**

(22) Date de dépôt : **05.07.79**

(54) **Procédé de préparation d'alpha aminoacides optiquement actifs et leurs dérivés.**

(30) Priorité : **07.07.78 FR 7820345**

(43) Date de publication de la demande : **06.02.80 Bulletin 80/03**

(45) Mention de la délivrance du brevet : **11.05.83 Bulletin 83/19**

(84) Etats contractants désignés : **CH DE GB NL**

(56) Documents cités :
**CHEM. PHARM. BULL., vol. 26, no. 3, mars 1978 Tokyo (JP) TOMEI OGURI et al. : « Amino acids and pertides XXIX A new efficient asymmetric synthesis of alpha-amino acid derivatives with recycle of a chiral reagent-asymmetric alkylation of a chiral schiff base from glycine » pages 803-808**
**BULLETIN DE LA SOCIETE CHIMIQUE DE FRANCE, 1968, no. 3 Paris (FR) J. MATHIEU : « La synthèse asymétrique au départ de carbones trigonaux » pages 1211-1244**

(73) Titulaire : **ANVAR Agence Nationale de Valorisation de la Recherche**
**13, rue Madeleine Michelis**
**F-92522 Neuilly-sur-Seine (FR)**

(72) Inventeur : **Duhamel, Lucette**
**32, rue Jacques Boutrolle**
**F-76130 Mont-Saint-Aignan (FR)**
Inventeur : **Plaquevent, Jean-Christophe**
**Rue Paul Painleve Imm. Bourbonnais no 37**
**F-76150 Maromme (FR)**

(74) Mandataire : **Nony, Michel**
**Cabinet Nony 29, rue Cambacérès**
**F-75008 Paris (FR)**

**EP 0 007 834 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

Procédé de préparation d'alpha-aminoacides optiquement actifs et leurs dérivés

La présente invention a pour objet un nouveau procédé de préparation d'alpha-aminoacides optiquement actifs, ou de leurs dérivés.

On sait que la préparation d'aminoacides optiquement actifs est intéressante car ceux-ci sont utilisés notamment dans les industries alimentaire et pharmaceutique.

On connaissait déjà la préparation d'aminoacides optiquement actifs par synthèse asymétrique. Ces procédés ont notamment fait l'objet d'une revue par K. WEINGES et B. STEMMLE, Recent Dev. Chem. Nat. Carbon Compd. 1976, *7*, 89.

Le procédé de la présente demande est fondé sur un principe différent et consiste à réaliser une déracémisation par protonation énantiosélective.

Le procédé de la présente demande est simple et rapide à mettre en œuvre. Il ne nécessite pas l'emploi de catalyseurs coûteux ou difficiles d'accès, et n'est pas limité aux substances présentant le phénomène de cristallisation isomérisante.

La présente invention a pour objet un procédé de préparation d'un alpha-aminoacide chiral ou d'un de ses dérivés, possédant un substituant hydrogène en $\alpha$ de la fonction carboxylique ou du dérivé de celle-ci, au départ de l'antipode optique correspondant ou d'un mélange dudit antipode optique avec ledit aminoacide chiral ou son dérivé, caractérisé par le fait que l'on soumet ledit produit de départ à l'action d'une base forte, afin d'éliminer le proton en $\alpha$ de la fonction carboxylique ou du dérivé de celle-ci, de façon connue en soi, puis que l'on fait agir sur le produit obtenu un agent de protonation chiral stériquement encombré.

On obtient ainsi, uniquement ou de façon prépondérante, un aminoacide ou son dérivé ayant une activité optique différente de celle du produit de départ. L'originalité de cette méthode réside donc essentiellement dans la possibilité théorique d'obtention d'un énantiomère pur à partir du mélange racémique ou même de son inverse optique, avec un rendement maximum pouvant atteindre 100 % alors qu'il est limité à 50 % dans le cas de la résolution.

Le produit de départ peut être par exemple un aminoacide (ou un de ses dérivés), généralement sous forme d'un mélange d'énantiomères, que l'on veut transformer, ou enrichir, en l'un des énantiomères. Parmi les aminoacides ou leurs dérivés utilisables comme produits de départ, on citera la phénylglycine, l'alanine, la phénylalanine, la valine, la leucine, l'isoleucine, la tyrosine, la sérine, la thréonine, la méthionine, le tryptophane, l'arginine, la lysine, l'histidine, etc..., et leurs dérivés.

Parmi les dérivés des aminoacides ou des mélanges d'énantiomères d'aminoacides de départ, on citera notamment les dérivés énolisables de la fonction carboxylique, tels que les esters, les thioesters, les nitriles, les amidines, les amides, etc..., ainsi que les dérivés de la fonction amine, tels que les bases de Schiff, les dérivés d'acylation, et plus généralement les dérivés d'aminoacides dans lesquels le groupement amine est transformé à l'aide d'un groupement protecteur pouvant être éliminé pour redonner l'amine non protégée.

De tels groupements protecteurs sont bien connus et sont décrits notamment par R.A. BOISSONNAS, Advances In Organic Chemistry, Methods and Results, Vol. 3, Interscience Publishers, 1963, pages 159 et suivantes, ainsi que par J.F.W. McOmie, idem, pages 191 et suivantes.

Parmi les groupements de protection temporaire de la fonction amine, on peut citer notamment les groupements carbo-tert-butyloxy, formyle, phtaloyle, etc... Bien entendu, les groupements réactifs, éventuellement présents, autres que le groupement $\alpha$-amino, peuvent être également protégés ; c'est le cas par exemple du groupement amine latérale de la lysine ou de l'ornithine, du groupe guanidino de l'arginine, des groupements hydroxyle de la sérine, de la thréonine, et de la tyrosine, du groupement thiol de la cystéine, etc...

Le produit de départ peut être par exemple une base de Schiff d'un ester d'un alpha-aminoacide. Ces bases de Schiff peuvent être obtenues selon des procédés connus, par exemple par l'action sur l'aminoacide du chlorure de thionyle, en présence d'un alcool, pour former le chlorhydrate d'aminoester correspondant, puis par l'action d'un dérivé carbonylé sur ledit chlorhydrate d'aminoester, dans les conditions usuelles de préparation des bases de Schiff.

Comme dérivé carbonylé, on utilise notamment ceux de formule $R_4$—CO—$R_3$, dans laquelle $R_3$ et $R_4$ peuvent représenter un groupement hydrocarboné éventuellement substitué, l'un d'entre eux pouvant en outre représenter un atome d'hydrogène.

Il convient de remarquer que l'expression « base de Schiff » s'étend ici, non seulement aux dérivés des aldéhydes aromatiques, mais plus généralement aux dérivés des cétones ou des aldéhydes aliphatiques ou aromatiques. Les substituants $R_3$ et $R_4$ peuvent représenter notamment des groupements aryle, alkyle linéaire ou ramifié, ou cycloalkyle, éventuellement substitués, cette dernière expression englobant les groupements comportant des hétéroatomes.

Généralement, les groupements hydrocarbonés que représentent $R_3$ et $R_4$ possèdent jusqu'à 20 atomes de carbone. Les substituants $R_3$ et $R_4$, lorsqu'ils représentent un groupement aryle, sont notamment un groupement phényle éventuellement substitué par un groupement alcoxy (tel que méthoxy ou éthoxy), alkyle (tel qu'isopropyle ou méthyle), etc...

Le produit de départ peut être soit un produit racémique, soit un mélange d'énantiomères en

proportions quelconques. Par le choix d'un acide chiral de configuration appropriée, il est possible soit d'obtenir un mélange enrichi en l'un des énantiomères dudit produit de départ, soit de transformer l'un des énantiomères en son antipode optique.

Il convient de noter que l'on utilise ici les expressions « énantiomères » et « antipode optique » par commodité, en faisant référence uniquement au carbone asymétrique en position α par rapport au carboxyle. Mais il est bien entendu que le procédé de la présente demande est applicable à des aminoacides possédant d'autres carbones asymétriques, en plus du carbone α.

Le procédé de l'invention est applicable notamment aux aminoacides, et à leurs dérivés, présentant le groupement caractéristique de formule I :

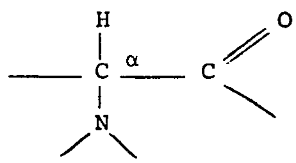

(I)

Les produits de formule I englobent notamment les dérivés de formule IA :

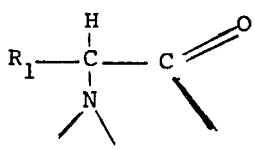

(IA)

dans laquelle $R_1$ est un groupement hydrocarboné, tel qu'un groupement alkyle, aryle ou aralkyle, éventuellement substitué et comportant éventuellement des hétéroatomes, et notamment un groupement phényle, benzyle, indole-3-yl méthyle, isopropyle, méthyle et 2-méthylpropyle.

La base forte utilisée dans le procédé défini ci-dessus est notamment une base permettant de transformer le groupement de formule I, par élimination d'un proton, en un énolate de formule II :

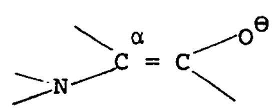

(II)

Parmi les bases fortes utilisables, on citera notamment les hydrures, les alcoolates, les amidures, notamment les hydrures, alcoolates ou amidures alcalins, par exemple les hydrures de sodium, de potassium ou de lithium, le terbutylate de lithium ou de potassium, le di-isopropylamidure de lithium, etc... On utilise généralement cette base forte à raison d'au moins un équivalent par mole de dérivé de formule I.

De plus, on a découvert que, de façon surprenante, le rendement optique augmente lorsque la base forte est elle-même porteuse d'une activité optique. Les bases fortes optiquement actives utilisables sont par exemple les hydrures, alcoolates et amidures (notamment les hydrures, alcoolates et amidures de métal alcalin), tels que ces bases portent un ou plusieurs atomes de carbone ou hétéroatomes asymétriques, ces groupements asymétriques conférant à la base utilisée une activité optique. Comme exemple de base optiquement active utilisable, on citera notamment un amidure, tel qu'un amidure alcalin, et en particulier l'amidure de lithium, de la (+) N-méthyl phényléthylamine.

La réaction d'énolisation est effectuée généralement dans un solvant, de préférence anhydre. Le solvant peut être notamment un éther tel que le tétrahydrofuranne ou l'éther éthylique, un alcool tel t-butanol, etc...

Les acides chiraux utilisables dans le procédé ci-dessus sont ceux qui possèdent un groupement stériquement encombrant.

Grâce à l'utilisation d'un tel acide, il est possible de fixer un proton de façon énantiosélective, sur le carbone α de l'énolate de formule II.

L'utilisation d'un acide chiral donné, stériquement encombré, permet la fixation préférentielle, sur l'une des faces du plan de la double liaison énolique, d'un proton, et l'utilisation de l'antipode optique de cet acide stériquement encombré, permet la fixation préférentielle d'un proton sur l'autre face du plan de la double liaison énolique.

Les acides chiraux stériquement encombrés sont ceux possédant au moins un substituant cyclique (saturé ou insaturé) ou un groupement ramifié. Les acides chiraux stériquement encombrés peuvent posséder notamment au moins un substituant alkyle, de préférence ramifié, éventuellement substitué ou

insaturé, cycloalkyle ou aryle, éventuellement substitué, ou un substituant hydrocarboné ponté, ou encore porter au moins un groupement alkyle en ramification.

Parmi les acides utilisables on citera notamment les acides diacyltartriques et leurs monoesters, l'acide camphanique, l'acide mandélique, l'acide 2-phtalimido 3-phényl propionique, l'acide 2-trifluorométhyl 2-méthoxy phénylacétique, etc...

Parmi les acides chiraux utilisables, on citera notamment les acides diacyl-tartriques de formule IV :

$$HO_2C—CH(—O—CO—R_5)—CH(—O—CO—R_5)—CO_2H \qquad (IV)$$

dans laquelle $R_5$ est un reste d'un acide carboxylique, de préférence encombré, et les monoesters des diacides de formule IV.

Le substituant $R_5$ peut être notamment un alkyle, de préférence ramifié, éventuellement substitué ou insaturé, un groupement cycloalkyle, un groupement aryle, notamment phényle ou naphtyle, éventuellement substitué, un groupement hydrocarboné ponté comme par exemple un groupement adamantyle, etc...

Parmi les acides diacyl-tartriques de formule IV, on citera notamment ceux qui sont mentionnés dans la partie expérimentale ci-après.

Tous ces acides encombrés sont connus ou peuvent être préparés selon les méthodes générales décrites dans la littérature.

Par exemple, pour préparer le di-adamantylcarboxylate de l'acide tartrique, on mélange 3 moles du chlorure de l'acide adamantylcarboxylique avec une mole d'acide tartrique. On chauffe à 140 °C pendant 2-3 heures, refroidit, et reprend par le benzène. On sépare l'anhydride qui cristallise, le reprend par l'acétone et ajoute la quantité stœchiométrique d'eau. On chauffe au reflux, puis évapore l'acétone. On obtient l'acide de formule IV (avec $R_5$ = adamantyle).

On utilise ces acides chiraux à raison d'au moins un équivalent. On utilise généralement un excès d'acide chiral, de préférence à raison de deux équivalents d'acide chiral pour une mole du dérivé de formule I.

On fait agir l'acide chiral en solution dans un solvant, ce solvant étant de préférence le même que celui utilisé dans la réaction d'énolisation.

De préférence on fait agir l'acide chiral à des températures pouvant varier de 0 à − 100 °C, et en particulier vers − 70 °C, pendant un temps pouvant varier de quelques minutes à quelques heures. Par exemple, la réaction de protonation est généralement terminée au bout de 15 minutes à − 70 °C.

On isole le dérivé d'aminoacide optiquement actif selon les méthodes connues. Par exemple, on sépare l'agent de protonation par formation quantitative d'un sel insoluble dans l'éther dans lequel la base de Schiff reste en solution.

Après la réaction de protonation, on peut transformer le produit obtenu en tout autre dérivé correspondant à la formule I, par les réactions connues de transformation, par exemple de déprotection, sans racémisation. Par exemple, on transforme une base de Schiff obtenue, de formule III :

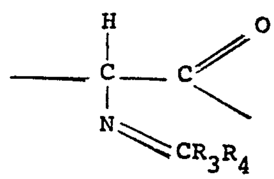

(III)

en aminoester ou en aminoacide correspondant selon les méthodes connues, par hydrolyse acide. L'aminoester peut être transformé en aminoacide par hydrolyse alcaline, etc.

En outre, il est possible de récupérer, avec un bon rendement, et sans racémisation, l'acide chiral tel que IV utilisé, dans la réaction de protonation. Un exemple détaillé est donné dans la partie expérimentale.

Les exemples suivants illustrent l'invention sans toutefois la limiter. Dans tous ces exemples, les manipulations sont effectuées sous atmosphère inerte, par exemple sous azote.

## Exemple 1

Protonation énantiosélective du N-benzylidène phénylglycinate de méthyle par l'acide L-dipivaloyltartrique (formule IV, $R_5$ = t-butyle)

On dissout 0,5 g ($5 \cdot 10^{-3}$M) de diisopropylamine dans 15 $cm^3$ de tétrahydrofuranne sec. On refroidit à − 5 °C et ajoute à cette température 3,12 $cm^3$ de n-butyllithium 1,6 M, en solution dans l'hexane ($5 \cdot 10^{-3}$M).

On agite la solution pendant 1/4 heure à une température comprise entre − 5 °C et 0 °C. On refroidit

alors à – 70 °C et ajoute à cette température 0,885 g ($3,5 \cdot 10^{-3}$M) de la base de Schiff de l'ester méthylique de la phénylglycine racémique, en solution dans 6 $cm^3$ de tétrahydrofuranne sec. On agite 1/4 heure à – 70 °C, puis ajoute 3,18 g ($1 \cdot 10^{-2}$M) d'acide L-dipivaloyltartrique en solution dans 10 $cm^3$ de tétrahydrofuranne sec. Après 1/4 heure d'agitation à – 70 °C, on laisse revenir à température ambiante. La solution est traitée par 30 $cm^3$ d'une solution aqueuse saturée de $NaHCO_3$ ; la phase organique est lavée par 30 $cm^3$ d'eau, séchée au sulfate de magnésium et concentrée sous pression réduite. L'huile jaune obtenue (base de Schiff correspondante optiquement active de la série L) est cristallisée dans l'hexane ; on filtre et sèche les cristaux. 0,75 g ; Rdt : 85 %.

$$(\alpha)_D^{25} = -41{,}8°.$$

Le rendement optique est de 50 %.

Récupération de l'acide L-dipivaloyl tartrique

La phase aqueuse est lavée 3 fois par 30 $cm^3$ d'éther, acidifiée jusqu'à pH 1 par une solution aqueuse à 10 % d'HCl. On extrait 3 fois par 30 $cm^3$ d'éther. La phase organique est séchée sur sulfate de magnésium, filtrée et le solvant est évaporé sous le vide de la trompe à eau. L'huile incolore obtenue est cristallisée dans le pentane. On filtre et sèche les cristaux dans un dessiccateur.

(Poids obtenu = 3,05 g ; Rdt : 96 %.)

Exemple 2

Protonations énantiosélectives de la base de Schiff de l'ester méthylique du tryptophane, de formule III,

avec $R_3 = H$ et $R_4 = C_6H_5$.

On procède de même que précédemment dans l'exemple 1, mais en ajoutant seulement $1,7 \cdot 10^{-3}$M de la base de Schiff racémique (0,52 g).

On obtient la base de Schiff optiquement active (Série L) avec un rendement optique de 30,5 %.

Exemple 3

On opère comme précédemment au départ des bases de Schiff (racémiques) de formule $III_x$ :

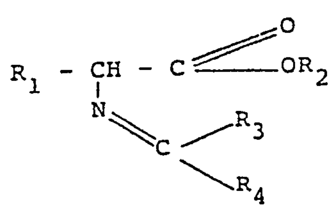

($III_x$)

dans laquelle $R_1$ est le reste d'un α-aminoacide de formule : $R_1$—$CH(NH_2)$—$CO_2H$, $R_1$ étant un groupement hydrocarboné, tel qu'un groupement alkyle, aryle, aralkyle, éventuellement substitué et comportant éventuellement des hétéroatomes, et notamment un groupement phényle, benzyle, indole-3-yl méthyle, isopropyle, méthyle et 2-méthylpropyle, $R_2$ est le reste d'un alcool $R_2$—OH, par exemple un reste alkyle, aralkyle, etc...

Les formules de quelques bases de Schiff utilisées dans cet exemple sont résumées dans le tableau 1 suivant ($R_3$ = H, $R_4$ = phényle, $R_2$ = méthyle).

Tableau 1

| Base de Schiff $III_x$ avec x = | $R_1$ | Caractéristiques du produit optiquement pur Série | $(\alpha)_D^{25}$ |
|---|---|---|---|
| 1 | phényle | D | + 84° (c = 3, $CHCl_3$) |
| 2 | benzyle | L | – 245° (c = 4, $CHCl_3$) |
| 3 | indole-3-yl méthyle | L | – 290° (c = 3, $CHCl_3$) |
| 4 | isopropyle | L | – 130° (c = 4, $CHCl_3$) |

Tableau 1 (suite)

| Base de Schiff $III_x$ avec x = | $R_1$ | Caractéristiques du produit optiquement pur Série | $(\alpha)_D^{25}$ |
|---|---|---|---|
| 5 | méthyle | L | -100° (liquide) |
| 6 | 2-méthylpropyle | | |

Les bases de Schiff précédentes ont été énolisées, puis protonées à l'aide des acides diacyltartriques (série L) suivants de formule IV (Bu signifie : butyle) :

Tableau 2

| Diacide de formule $IV_x$ avec x = | $R_5$ |
|---|---|
| 1 | t-Bu |
| 2 | $t-Bu-CH_2-$ |
| 3 | $t-Bu-(CH_2)_2-$ |
| 4 | phényle |
| 5 | benzyle |
| 6 | phénéthyle |
| 7 | $(CH_3)_2-C\ Br-$ |
| 8 | cyclohexyle |
| 9 | $C_6H_5-CH=CH-$ |
| 10 | Adamantyle |
| 11 | méthyle |
| 12 | isopropyle |
| 13 | n-propyle |
| 14 | $(C_2H_5)_3C-$ |
| 15 | 1,1,3,3-tétraméthyl butyle |
| 16 | 2,4,6-triméthylphényle |
| 17 | α-naphtyle |
| 18 | o-tolyle |
| 19 | m-tolyle |
| 20 | 2,5-diméthyl phényl |

Les résultats obtenus avec ces acides sont résumés dans le tableau 3 suivant :

(Voir tableau 3, page 7)

Tableau 3

| Base de Schiff | Acide $IV_x$ avec x = | Rdt optique | Rdt chimique |
|---|---|---|---|
| | 2 | 16,3 | |
| | 3 | 33,5 | |
| | 4 | 12,3 | |
| | 5 | 8,25 | 80% |
| | 6 | 6,4 | |
| | 7 | 34,4 | |
| $III_1$ | 8 | 35,8 | à |
| | 9 | 25,1 | |
| | 10 | 53,2 | |
| | 11 | 2,6 | 85% |
| | 12 | 12,3 | |
| | 13 | 4,25 | |
| | 14 | 5,45 | |
| | 15 | 11,1 | |
| | 16 | 8,5 | |
| | 17 | 4,5 | |
| | 1 | 27 | |
| | 2 | 8 | 60% |
| | 3 | 17,7 | |
| $III_2$ | 4 | 33,5 | à |
| | 5 | 15 | |
| | 6 | 3 | |
| | 20 | 18,5 | 65% |
| | 2 | 10,7 | 90% |
| $III_3$ | 3 | 14,6 | à |
| | 4 | 12,6 | 95% |
| | 5 | 3,8 | |
| | 6 | 7 | |
| | 1 | 33,5* | |
| | 2 | 21 | 70% |
| $III_4$ | 4 | 38,5* | |
| | 8 | 35 | à |
| | 9 | 20,5 | |
| | 18 | 24,5 | 75% |
| | 19 | 29 | |

Tableau 3 (suite)

| | | | |
|---|---|---|---|
| | 4 | 30 | 25% |
| $III_5$ | 19 | 28 | 60% |
| $III_6$ | 1 | 33,3* | 70% |

tous les produits obtenus sont de la série L.

* : Résultat confirmé ou déterminé par RMN, à l'aide d'un sel chiral d'europium.

## Exemple 4

Les bases de Schiff racémiques $III_1$-$III_4$ (voir exemple précédent) ont été énolisées, puis protonées comme précédemment avec divers acides optiquement actifs encombrés. Les résultats sont résumés dans le tableau 4 suivant :

Tableau 4

| Base de Schiff | Acide | Rdt optique % | Série | Rdt chimique % |
|---|---|---|---|---|
| $III_1$ | (−)camphanique | 2,7 | D | 80 |
| | (+)mandélique | 2,48 | D | 85 |
| | 2-phtalimido 3-phényl propionique (L) | 3,9 | L | 80 |
| | (−) 2-méthoxy 2-trifluoro méthyl phényl acétique | 14,5 | L | 85 |
| | (L) $t\text{-}Bu\text{-}CO_2\text{-}CH\text{-}CO_2H$ $t\text{-}Bu\text{-}CO_2$ $CH\text{-}CO_2CH_3$ | 19,6 | L | 80 |
| $III_2$ | (+)mandélique | 8,6 | D | 60 |
| $III_3$ | (+) mandélique | 8 | D | 90 |
| $III_4$ | (+) mandélique | 15,1 | D | 75 |

## Exemple 5

La base de Schiff racémique de formule :

$$C_6H_5{-}CH({-}N = CH{-}t{-}Bu){-}COOCH_3$$

a été énolisée, puis protonée comme précédemment avec les acides $IV_1$, $IV_3$ et $IV_4$.

Par hydrolyse chlorhydrique de la base de Schiff optiquement active résultante, on a obtenu le chlorhydrate d'aminoester optiquement actif de la série L, de formule :

$$C_6H_5{-}CH{-}COOCH_3$$

$$NH_3^{\oplus}Cl^{\ominus}$$

avec des rendements optiques de 8,5-7,5-4,7 % respectivement.

(Rdts chimiques : 60-65 %).

Exemple 6

On énolise la base de Schiff de l'ester méthylique de la phénylglycine (Formule III, avec $R_3$ = H et $R_4$ = phényl) par l'amidure de lithium de la (+) phényléthylamine N-méthylée, puis protoné l'énolate de lithium obtenu par l'acide (L) dipivaloyltartrique. On isole alors la base de Schiff de l'ester méthylique de la phénylglycine avec un rendement de 75 %.

Après purification par l'hexane, on obtient 60 % de produit dont l'hydrolyse acide conduit au chlorhydrate d'aminoester correspondant de rotation.

$$(\alpha)_D^{25} = +92{,}4° \ (CH_3OH)$$

Rendement optique : 70,5 %.

Rendement de l'hydrolyse : 95 %.

Réf : (D) Hydrochlorure de phénylglycinate de méthyle

$$(\alpha)_D^{25} = -131° \ (CH_3OH).$$

**Revendications**

1. Procédé de préparation d'un alpha-aminoacide chiral ou d'un de ses dérivés, possédant un substituant hydrogène en α de la fonction carboxylique ou du dérivé de celle-ci, au départ de l'antipode optique correspondant ou d'un mélange dudit antipode optique avec ledit aminoacide chiral ou son dérivé, caractérisé par le fait que l'on soumet ledit produit de départ à l'action d'une base forte, afin d'éliminer le proton en α de la fonction carboxylique ou du dérivé de celle-ci, de façon connue en soi, puis que l'on fait agir sur le produit obtenu un agent de protonation chiral stériquement encombré constitué par un acide chiral possédant au moins un substituant cyclique ou un groupement en ramification.

2. Procédé selon la revendication 1, caractérisé par le fait que ledit aminoacide ou son dérivé est choisi dans le groupe constitué par : la phénylglycine, l'alanine, la phénylalanine, la valine, la leucine, l'isoleucine, la tyrosine, la sérine, la thréonine, la méthionine, le tryptophane, l'arginine, la lysine et l'histidine, ainsi que leurs dérivés.

3. Procédé selon l'une quelconque des revendications précédentes, caractérisé par le fait que les dérivés de l'aminoacide ou du mélange d'aminoacides sont choisis dans le groupe constitué par les dérivés énolisables de la fonction carboxylique, tels que les esters, les thioesters, les nitriles, les amidines et les amides, et les dérivés de la fonction amine dans lesquels le groupement amine est transformé à l'aide d'un groupement protecteur, tels que les bases de Schiff et les dérivés d'acylation.

4. Procédé selon la revendication 3, caractérisé par le fait que le produit de départ est une base de Schiff de l'alpha-aminoacide ou d'un de ses dérivés.

5. Procédé selon la revendication 4, caractérisé par le fait que ladite base de Schiff résulte de l'action sur l'alphaaminoacide ou de son dérivé d'un composé carbonylé de formule $R_4$—CO—$R_3$, dans laquelle $R_3$ et $R_4$ représentent un radical hydrocarboné éventuellement substitué, l'un d'entre eux pouvant en outre représenter un atome d'hydrogène.

6. Procédé selon la revendication 5, caractérisé par le fait que les substituants $R_3$ et $R_4$, lorsqu'ils représentent un radical hydrocarboné, éventuellement substitué, représentent un groupement aryle, alkyle linéaire ou ramifié, ou cyclo-alkyle, éventuellement substitué, contenant éventuellement des hétéroatomes.

7. Procédé selon l'une quelconque des revendications 5 et 6, caractérisé par le fait que le produit de départ est une base de Schiff de formule :

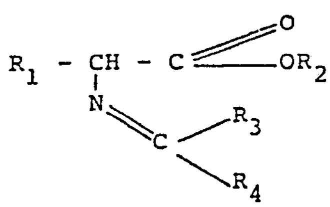

dans laquelle $R_1$ est le reste d'un α-aminoacide de formule : $R_1$—CH($NH_2$)—$CO_2H$, $R_2$ est le reste d'un alcool de formule $R_2$—OH, dans lesquelles $R_1$ représente un groupement hydrocarboné, tel qu'un groupement alkyle, aryle ou aralkyle, éventuellement substitué et comportant éventuellement des hétéroatomes, $R_2$ un reste alkyle ou aralkyle, et $R_3$ et $R_4$ sont définis comme précédemment.

8. Procédé selon la revendication 7, caractérisé par le fait que $R_1$ représente un groupement

phényle, benzyle, indole-3-yl méthyle, isopropyle, méthyle ou 2-méthylpropyle.

9. Procédé selon l'une quelconque des revendications précédentes, caractérisé par le fait que la base forte est choisie dans le groupe constitué par les hydrures, les alcoolates et les amidures.

10. Procédé selon la revendication 9, caractérisé par le fait que la base forte est utilisée à raison d'au moins un équivalent par mole d'aminoacide, ou de son dérivé, de départ.

11. Procédé selon l'une quelconque des revendications précédentes, caractérisé par le fait que la réaction avec la base forte est effectuée dans un solvant anhydre.

12. Procédé selon la revendication 11, caractérisé par le fait que ledit solvant est un éther tel que le tétrahydrofuranne ou l'éther éthylique ou un alcool tel que le t-butanol.

13. Procédé selon l'une quelconque des revendications 1 à 12, caractérisé par le fait que l'acide chiral stériquement encombré possède au moins un substituant alkyle, de préférence ramifié, éventuellement substitué ou insaturé, cycloalkyle ou aryle, éventuellement substitué, ou un substituant hydrocarboné ponté, ou porte au moins un groupement alkyle en ramification.

14. Procédé selon la revendication 13, caractérisé par le fait que l'acide chiral est choisi dans le groupe constitué par les acides diacyltartriques et leurs monoesters, l'acide camphanique, l'acide mandélique, l'acide 2-phtalimido 3-phényl propionique, et l'acide 2-trifluorométhyl 2-méthoxy phénylacétique.

15. Procédé selon la revendication 14, caractérisé par le fait que lesdits acides chiraux sont les acides diacyltartriques de formule :

$$HO_2C—CH\ (—O—CO—R_5)—CH\ (—O—CO—R_5)—CO_2H$$

dans laquelle $R_5$ est le reste d'un acide carboxylique, de préférence encombré, ou les monoesters de ces diacides.

16. Procédé selon la revendication 15, caractérisé par le fait que $R_5$ est un groupement alkyle de préférence ramifié, éventuellement substitué ou insaturé, un groupement aryle, tel qu'un groupement phényle ou naphtyle, éventuellement substitué, ou un groupement hydrocarboné ponté tel qu'un groupement adamantyle.

17. Procédé selon la revendication 16, caractérisé par le fait que $R_5$ représente un groupement méthyle, n-propyle, isopropyle, t-butyle, néopentyle, 3,3-diméthylbutyle, phényle, benzyle, phénéthyle, $(CH_3)_2$—CBr—, cyclohexyle, styryle, adamantyle, $(C_2H_5)_3C$—, 1,1,3,3-tétraméthyl butyle, 2,4,6-triméthylphényle, α-naphtyle, o-tolyle, m-tolyle ou 2,5-diméthyl phényle.

18. Procédé selon l'une quelconque des revendications précédentes, caractérisé par le fait que l'on fait agir ledit acide chiral à raison d'au moins un équivalent pour une mole du dérivé d'aminoacide.

19. Procédé selon l'une quelconque des revendications précédentes, caractérisé par le fait que l'on fait agir ledit acide chiral en solution dans un solvant, tel que le solvant utilisé dans la réaction d'énolisation.

20. Procédé selon l'une quelconque des revendications précédentes, caractérisé par le fait que l'on fait agir ledit acide chiral à une température pouvant varier de 0 à − 100 °C.

21. Procédé selon la revendication 20, caractérisé par le fait que l'on fait agir l'acide chiral à température de − 70 °C environ.

22. Procédé selon l'une quelconque des revendications 9 à 21, caractérisé par le fait que ladite base forte est porteuse d'une activité optique.

23. Procédé selon la revendication 22, caractérisé par le fait que ladite base forte est un amidure de la (+) N-méthyl phényléthylamine.

## Claims

1. Process for the preparation of a chiral alpha-aminoacid or of one of its derivatives, possessing a hydrogen substituent in the α-position to the carboxyl group or to the derivative of the latter, starting from the corresponding optical antipode or from a mixture of the said optical antipode with the said chiral aminoacid or its derivative, characterised in that the said starting material is subjected to the action of a strong base in order to remove the proton in the α-position to the carboxyl group or to the derivative of the latter, in a manner which is in itself known, and then in that a sterically hindered, chiral protonating agent, consisting of a chiral acid possessing at least one cyclic substituent or one branched group, is reacted with the product obtained.

2. Process according to Claim 1, characterised in that the said aminoacid or its derivative is chosen from the group comprising : phenylglycine, alanine, phenylalanine, valine, leucine, isoleucine, tyrosine, serine, threonine, methionine, tryptophan, arginine, lysine and histidine, and also derivatives thereof.

3. Process according to either one of the preceding claims, characterised in that the derivatives of the aminoacid or of the mixture of aminoacids are chosen from the group comprising enolisable derivatives of the carboxyl group, such as esters, thioesters, nitriles, amidines and amides, and derivatives of the amine group in which the amine group is converted by means of a protecting group, such as Schiff bases and acylation derivatives.

4. Process according to Claim 3, characterised in that the starting material is a Schiff base of the alpha-aminoacid or of one of its derivatives.

5. Process according to Claim 4, characterised in that the said Schiff base results from the action, on the alpha-aminoacid or its derivative, of a carbonyl compound of the formula $R_4$—CO—$R_3$, in which $R_3$ and $R_4$ represent an optionally substituted hydrocarbon radical, it also being possible for one of them to represent a hydrogen atom.

6. Process according to Claim 5, characterised in that, when they represent an optionally substituted hydrocarbon radical, the substituents $R_3$ and $R_4$ represent an aryl, linear or branched alkyl or cycloalkyl group which is optionally substituted and optionally contains hetero-atoms.

7. Process according to either one of Claims 5 and 6, characterised in that the starting material is a Schiff base of the formula :

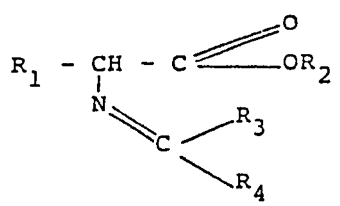

in which $R_1$ is the radical of an α-aminoacid of the formula : $R_1$—CH($NH_2$)—$CO_2H$, $R_2$ is the radical of an alcohol of the formula $R_2$—OH, in which formulae $R_1$ represents a hydrocarbon group, such as an alkyl, aryl or aralkyl group, which is optionally substituted and optionally contains hetero-atoms, and $R_2$ represents an alkyl or aralkyl radical, and $R_3$ and $R_4$ are defined as above.

8. Process according to Claim 7, characterised in that $R_1$ represents a phenyl, benzyl, indol-3-yl-methyl, isopropyl, methyl or 2-methylpropyl group.

9. Process according to any one of the preceding claims, characterised in that the strong base is chosen from the group comprising hydrides, alcoholates and metal amides.

10. Process according to Claim 9, characterised in that the strong base is used in an amount of at least one equivalent per mol of starting aminoacid or of its derivative.

11. Process according to any one of the preceding claims, characterised in that the reaction with the strong base is carried out in an anhydrous solvent.

12. Process according to Claim 11, characterised in that the said solvent is an ether, such as tetrahydrofuran or ethyl ether, or an alcohol, such as t-butanol.

13. Process according to any one of Claims 1 to 12, characterised in that the sterically hindered, chiral acid possesses at least one optionally substituted or unsaturated, preferably branched alkyl substituent, one optionally substituted cycloalkyl or aryl substituent or one bridged hydrocarbon substituent, or carries at least one alkyl group as a branch.

14. Process according to Claim 13, characterised in that the chiral acid is chosen from the group comprising diacyltartaric acids and their monoesters, camphanic acid, mandelic acid, 2-phthalimido-3-phenylpropionic acid and 2-trifluoromethyl-2-methoxyphenylacetic acid.

15. Process according to Claim 14, characterised in that the said chiral acids are the diacyltartaric acids of the formula :

$$HO_2C—CH(—O—CO—R_5)—CH(—O—CO—R_5)—CO_2H$$

in which $R_5$ is the radical of a preferably hindered carboxylic acid, or monoesters of these diacids.

16. Process according to Claim 15, characterised in that $R_5$ is an optionally substituted or unsaturated, preferably branched alkyl group, an aryl group, such as a phenyl or naphthyl group, which is optionally substituted, or a bridged hydrocarbon group such as an adamantyl group.

17. Process according to Claim 16, characterised in that $R_5$ represents a methyl, n-propyl, isopropyl, t-butyl, neopentyl, 3,3-dimethylbutyl, phenyl, benzyl, phenethyl, $(CH_3)_2$—CBr—, cyclohexyl, styryl, adamantyl, $(C_2H_5)_3C$—, 1,1,3,3-tetramethylbutyl, 2,4,6-trimethylphenyl, α-naphthyl, o-tolyl, m-tolyl or 2,5-dimethylphenyl group.

18. Process according to any one of the preceding claims, characterised in that the said chiral acid is reacted in an amount of at least one equivalent per mol of the aminoacid derivative.

19. Process according to any one of the preceding claims, characterised in that the said chiral acid is reacted in solution in a solvent, such as the solvent used in the enolisation reaction.

20. Process according to any one of the preceding claims, characterised in that the said chiral acid is reacted at a temperature which can vary from 0 to − 100 °C.

21. Process according to Claim 20, characterised in that the chiral acid is reacted at a temperature of about − 70 °C.

22. Process according to any one of Claims 9 to 21, characterised in that the said strong base is optically active.

23. Process according to Claim 22, characterised in that the said strong base is a metal amide of (+)-N-methylphenylethylamine.

**Ansprüche**

1. Verfahren zur Herstellung einer chiralen α-Aminosäure oder eines ihrer Derivate, die in α-Stellung zur Carboxylfunktion oder eines Derivates davon einen Wasserstoffsubstituenten besitzt, ausgehend von dem entsprechenden optischen Antipoden oder von einer Mischung dieses optischen Antipoden mit der chiralen Aminosäure oder ihres Derivates, dadurch gekennzeichnet, daß man das Ausgangsprodukt mit einer starken Base behandelt, um in an sich bekannter Weise das Proton in α-Stellung zur Carboxylfunktion oder eines Derivates davon abzuspalten und dann das erhaltene Produkt mit einem sterisch sperrigen, chiralen Protonierungsmittel, bestehend aus einer chiralen Säure mit wenigstens einem cyclischen Substituenten oder einer verzweigten Gruppe, umsetzt.

2. Verfahren gemäß Anspruch 1, dadurch gekennzeichnet, daß die Aminosäure oder ihr Derivat ausgewählt ist unter Phenylglycin, Alanin, Phenylalanin, Valin, Leucin, Isoleucin, Tyrosin, Serin, Threonin, Methionin, Tryptophan, Arginin, Lysin und Histidin, sowie deren Derivaten.

3. Verfahren gemäß einem der vorhergehenden Ansprüche, dadurch gekennzeichnet, daß die Derivate der Aminosäure oder der Mischung von Aminosäuren ausgewählt sind unter den enolisierbaren Derivaten der Carboxylfunktion, wie den Estern, Thioestern, Nitrilen, Amidinen und Amiden, und unter den Derivaten der Aminfunktion, bei denen die Aminogruppe durch eine Schutzgruppe abgewandelt ist, wie den Schiff'schen Basen und den Acylierungsderivaten.

4. Verfahren gemäß Anspruch 3, dadurch gekennzeichnet, daß das Ausgangsprodukt eine Schiff'sche Base der α-Aminosäure oder eines ihrer Derivate ist.

5. Verfahren gemäß Anspruch 4, dadurch gekennzeichnet, daß die Schiff'sche Base erhalten wird durch Umsetzung der α-Aminosäure oder ihres Derivates mit einer Carbonylverbindung der Formel $R_4$—CO—$R_3$, wobei $R_3$ und $R_4$ einen gegebenenfalls substituierten Kohlenwasserstoffrest bedeuten und einer der beiden Reste außerdem ein Wasserstoffatom bedeuten kann.

6. Verfahren gemäß Anspruch 5, dadurch gekennzeichnet, daß die Substituenten $R_3$ und $R_4$, wenn sie einen gegebenenfalls substituierten Kohlenwasserstoffrest bedeuten, für eine Aryl-, lineare oder verzweigte Alkyl-, oder Cycloalkylgruppe, die gegebenenfalls substituiert sind und die gegebenenfalls Heteroatome enthalten, stehen.

7. Verfahren gemäß einem der Ansprüche 5 und 6, dadurch gekennzeichnet, daß das Ausgangsprodukt eine Schiff'sche Base der Formel :

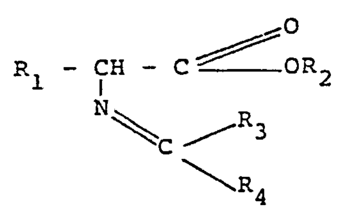

ist, worin $R_1$ einen α-Aminosäurerest der Formel $R_1$—CH($NH_2$)—$CO_2H$ bedeutet, $R_2$ den Rest eines Alkohols der Formel $R_2$—OH bedeutet, wobei $R_1$ für einen gegebenenfalls substituierten und gegebenenfalls Heteroatome enthaltenden Kohlenwasserstoffrest, wie eine Alkyl-, Aryl- oder Aralkylgruppe, steht, $R_2$ einen Alkyl- oder Aralkylrest bedeutet und $R_3$ und $R_4$ wie zuvor definiert sind.

8. Verfahren gemäß Anspruch 7, dadurch gekennzeichnet, daß $R_1$ eine Phenyl-, Benzyl-, Indol-3-yl-methyl-, Isopropyl-, Methyl- oder 2-Methylpropylgruppe bedeutet.

9. Verfahren gemäß einem der vorhergehenden Ansprüche, dadurch gekennzeichnet, daß die starke Base ausgewählt ist unter Hydriden, Alkoholaten und Amiden.

10. Verfahren gemäß Anspruch 9, dadurch gekennzeichnet, daß die starke Base in einem Verhältnis von wenigstens einem Äquivalent pro Mol der als Ausgangsprodukt eingesetzten Aminosäure oder ihres Derivats verwendet wird.

11. Verfahren gemäß einem der vorhergehenden Ansprüche, dadurch gekennzeichnet, daß die Umsetzung mit der starken Base in einem wasserfreien Solvens durchgeführt wird.

12. Verfahren gemäß Anspruch 11, dadurch gekennzeichnet, daß das Solvens ein Äther, wie Tetrahydrofuran oder Äthyläther, oder ein Alkohol, wie tert.-Butanol, ist.

13. Verfahren gemäß einem der Ansprüche 1 bis 12, dadurch gekennzeichnet, daß die chirale, sterisch sperrige Säure wenigstens einen, vorzugsweise verzweigten, gegebenenfalls substituierten oder ungesättigten Alkyl-, gegebenenfalls substituierten Cycloalkyl- oder Arylsubstituenten oder einen verbrückten Kohlenwasserstoffsubstituenten besitzt oder wenigstens eine verzweigte Alkylgruppe trägt.

14. Verfahren gemäß Anspruch 13, dadurch gekennzeichnet, daß die chiralen Säure ausgewählt ist unter Diacylweinsäuren und ihren Monoestern, Camphansäure, Mandelsäure, 2-Phthalimido-3-phenyl-

propionsäure und 2-Trifluormethyl-2-methoxyphenylessigsäure.

15. Verfahren gemäß Anspruch 14, dadurch gekennzeichnet, daß die chiralen Säuren Diacylweinsäuren der Formel :

$$HO_2C—CH(—O—CO—R_5)—CH(—O—CO—R_5)—CO_2H$$

worin $R_5$ einen vorzugsweise sperrigen Carbonsäurerest bedeutet, oder Monoester dieser Disäuren sind.

16. Verfahren gemäß Anspruch 15, dadurch gekennzeichnet, daß $R_5$ eine vorzugsweise verzweigte, gegebenenfalls substituierte oder ungesättigte Alkylgruppe, eine gegebenenfalls substituierte Arylgruppe, wie eine Phenyl- oder Naphtylgruppe, oder eine verbrückte Kohlenwasserstoffgruppe, wie eine Adamantylgruppe, ist.

17. Verfahren gemäß Anspruch 16, dadurch gekennzeichnet, daß $R_5$ eine Methyl-, n-Propyl-, Isopropyl-, tert.-Butyl-, Neopentyl-, 3,3-Dimethylbutyl-, Phenyl-, Benzyl-, Phenyläthyl-, $(CH_3)_2$—CBr—, Cyclohexyl-, Styryl-, Adamantyl-, $(C_2H_5)_3C$—, 1,1,3,3-Tetramethylbutyl-, 2,4,6-Trimethylphenyl-, α-Naphthylo-Tolyl-, m-Tolyl- oder 2,5-Dimethylphenylgruppe darstellt.

18. Verfahren gemäß einem der vorhergehenden Ansprüche, dadurch gekennzeichnet, daß man die chirale Säure in einem Verhältnis von wenigstens einem Äquivalent pro einem Mol Aminosäurederivat einsetzt.

19. Verfaren gemäß einem der vorhergehenden Ansprüche, dadurch gekennzeichnet, daß man die chirale Säure gelöst in einem Solvens, wie dem bei der Enolisierungsreaktion verwendeten Solvens, reagieren läßt.

20. Verfahren gemäß einem der vorhergehenden Ansprüche, dadurch gekennzeichnet, daß man die chirale Säure bei einer Temperatur, die zwischen 0 und – 100 °C variieren kann, reagieren läßt.

21. Verfahren gemäß Anspruch 20, dadurch gekennzeichnet, daß man die chirale Säure bei einer Temperatur von ungefähr – 70 °C reagieren läßt.

22. Verfahren gemäß einem der Ansprüche 9 bis 21, dadurch gekennzeichnet, daß die starke Base Trägerin einer optischen Aktivität ist.

23. Verfahren gemäß Anspruch 22, dadurch gekennzeichnet, daß die starke Base ein Amid des (+)-N-Methyl-phenyläthylamins ist.